(19) Europäisches Patentamt
European Patent Office
Office européen des brevets

(11) Veröffentlichungsnummer : **0 083 764**
**B1**

(12) # EUROPÄISCHE PATENTSCHRIFT

(45) Veröffentlichungstag der Patentschrift : **05.03.86**

(51) Int. Cl.[4] : **C 07 C125/04**

(21) Anmeldenummer : **82111705.8**

(22) Anmeldetag : **16.12.82**

(54) **Verfahren zur Herstellung von Carbamaten.**

(30) Priorität : **12.01.82 DE 3200559**

(43) Veröffentlichungstag der Anmeldung : **20.07.83 Patentblatt 83/29**

(45) Bekanntmachung des Hinweises auf die Patenterteilung : **05.03.86 Patentblatt 86/10**

(84) Benannte Vertragsstaaten : **BE CH DE FR GB LI NL**

(56) Entgegenhaltungen :
**DE-A- 2 459 765**
**DE-B- 1 198 344**
**DE-B- 1 643 635**
**GB-A- 982 785**
**US-A- 2 837 561**

(73) Patentinhaber : **BASF Aktiengesellschaft**
**Carl-Bosch-Strasse 38**
**D-6700 Ludwigshafen (DE)**

(72) Erfinder : **Mattner, Otto**
**Wormser Strasse 27A**
**D-6720 Speyer (DE)**
Erfinder : **Merger, Franz, Dr.**
**Max-Slevogt-Strasse 25**
**D-6710 Frankenthal (DE)**
Erfinder : **Nestler, Gerhard, Dr.**
**Van-Leyden-Strasse 17**
**D-6700 Ludwigshafen (DE)**
Erfinder : **Towae, Friedrich, Dr.**
**Parkstrasse 22**
**D-6700 Ludwigshafen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 083 764 B1

Jouve, 18, rue St-Denis, 75001 Paris, France

**Beschreibung**

Die Erfindung betrifft ein Verfahren zur Herstellung von Carbamaten durch Umsetzung von Harnstoff mit Alkoholen bei erhöhter Temperatur und unter Druck, wobei Inertgas durch das Reaktionsgemisch geleitet und der Druck während der Umsetzung so eingestellt wird, daß das Reaktionsgemisch siedet.

Aus Houben-Weyl, Methoden der Organischen Chemie, Band 8, Seiten 140-141, ist bekannt, daß man Harnstoff mit Alkoholen zu Carbamaten umsetzen kann. Die Umsetzung erfolgt drucklos am Siedepunkt des Gemisches. Es wird im Falle des Butylesters nur eine Ausbeute von 75 % erzielt. Ein weiteres Beispiel mit Zinkacetat oder Stannichlorid als Katalysator zeigt, wenn auch für den Fall des Benzylesters, eine wesentlich bessere Ausbeute (87 bzw. 92 %). Nach Paquin (Z. f. Naturforschung *1*, Seiten 518-523) kann durch Zusatz von Schwermetallsalzen die Ausbeute gesteigert werden. Der Nachteil dieser Methoden besteht jedoch darin, daß verschiedene Nebenprodukte, z. B. Biuret, Allophansäureester, Cyansäure bzw. Carbonate in beträchtlichen Mengen entstehen und die Ausbeute entsprechend schlecht ist (DE-OS-2 459 765).

Um die Bildung dieser Nebenprodukte zu verhindern, wird in der DE-OS 1 643 635 die Verwendung von Nickelsalzen als Katalysatoren vorgeschlagen. Es lassen sich nach dieser Methode zwar Ausbeuten von 94 % erzielen, doch lange Reaktionszeiten und die abwasserbelastende Entfernung der cancerogenen Nickelsalze aus dem Reaktionsgemisch bzw. die Aufarbeitung der entsprechenden Abwässer haben dazu geführt, daß dieses Verfahren keine wirtschaftliche Bedeutung erlangt hat. In der DE-OS-2 459 765 wird nun die Verwendung von Nickel enthaltenden Ionenaustauschern als Katalysatoren vorgeschlagen. Nachteilig bei diesem Verfahren ist jedoch wieder das Arbeiten mit cancerogenen Nickelsalzen, die aufwendige und abwasserbelastende Herstellung des Katalysators (DE-OS-2 459 765 Beispiel 1a), die Bildung von Carbonaten und die im Reaktionsgemisch gelösten Nickelverbindungen, die aufgrund ihrer Toxizität entfernt werden müssen.

Die GB-A-982 785 lehrt ein Verfahren zur Herstellung von Carbamaten durch Umsetzung von Harnstoff mit Alkoholen ohne Verwendung eines Katalysators bei Temperaturen von 180 bis 230 °C, wobei stets soviel des entstehenden Ammoniakgases im System gehalten wird, daß das Reaktionsgemisch siedet. Nach diesem Verfahren fallen die Carbamate bei diskontinuierlicher Fahrweise in Ausbeuten von nur 82 bis 92 %, bezogen auf den eingesetzten Harnstoff, an. Die Produkte müssen entweder durch aufwendige Vakuumdestillation oder durch Kristallisation gereinigt werden.

Es wurde nun gefunden, daß man Carbamate der Formel

$$R^1 - O - \overset{\overset{O}{\parallel}}{C} - NH_2 \qquad (I)$$

worin $R^1$ einen aliphatischen, cycloaliphatischen, araliphatischen Rest oder den Rest $R^3—(OR^2)_n—$ bedeutet, $R^2$ einen aliphatischen Rest mit mindestens 2 Kohlenstoffatomen bedeutet, $R^3$ für einen aliphatischen, cycloaliphatischen oder araliphatischen Rest steht, und n eine ganze Zahl bezeichnet, durch Umsetzung von Harnstoff mit Alkoholen vorteilhaft erhält, wenn man Harnstoff mit Alkoholen der Formel

$$R^1—OH \qquad (II)$$

worin $R^1$ die vorgenannte Bedeutung besitzt, bei einer Temperatur von mindestens 140 °C und einem Druck von mindestens 2 bar umsetzt, wobei Inertgas durch das Reaktionsgemisch geleitet und der Druck während der Umsetzung so eingestellt wird, daß das Reaktionsgemisch siedet.

Das Verfahren wird vorzugsweise ohne Verwendung eines Katalysators durchgeführt.

Die Umsetzung kann für den Fall der Verwendung von Methylglykol durch die folgenden Formeln wiedergegeben werden :

$$CH_3OCH_2CH_2OH + H_2NCONH_2 \rightarrow CH_3OCH_2CH_2OCONH_2 + NH_3$$

Im Vergleich zu dem bekannten Verfahren liefert das Verfahren nach der Erfindung auf einfacherem und wirtschaftlicherem Wege Carbamate in besserer Ausbeute, Raum-Zeit-Ausbeute und Reinheit. Es bietet im Vergleich zu dem in der deutschen Offenlegungsschrift 1 643 635 beschriebenen Verfahren technische Vorteile in Gestalt wesentlich verringerter Reaktionszeit von im allgemeinen 5 bis 9 Stunden und der erhöhten Raum-Zeit-Ausbeute. Es war aufgrund vorgenannter Veröffentlichungen überraschend, daß das Verfahren nach der Erfindung ohne Verwendung von Katalysatoren Carbamate auf einfachem Wege in besserer Ausbeute und Reinheit ohne wesentliche Bildung von Nebenprodukten liefert. Die so erhaltenen Carbamate genügen höchsten Anforderungen der Reinheit, sie können z. B. mit Formaldehyd ohne vorherige Reinigung zu für die chlorechte Textilausrüstung verwendeten Dimethylolverbindungen umgesetzt werden und ermöglichen somit einen arbeitssparenderen und wirtschaftlicheren Weg für die Herstellung von Hilfsmitteln, z. B. Textilausrüstungs- und Textilhochveredlungsmitteln. Man hätte insbesondere im Hinblick auf die deutsche Offenlegungsschrift 1 643 635 zumindest keine besseren

Ausbeuten und bessere Reinheit und sogar eine Verschlechterung der Ergebnisse erwarten müssen, da entgegen der Lehre der Offenlegungsschrift, nur Nickelsalze und diese in einer Menge von 0,1 bis 5 Gew.%, bezogen auf Harnstoff, zu verwenden, und entgegen der Lehre der DE-OS-2 459 765 Kationenaustauscher und den als Kation an den Austauscher gebundenen Nickel zu verwenden, das erfindungsgemäße Verfahren ohne Katalysatoren durchgeführt wird. Durch den Wegfall von Katalysatoren und Austauschern ist das erfindungsgemaße Verfahren betriebssicherer, vermeidet toxische Produkte und Abwasserprobleme und ist somit umweltfreundlicher.

Bevorzugte Ausgangsstoffe II und dementsprechend bevorzugte Endstoffe I sind solche, in deren Formeln $R^1$ einen Alkylrest mit 1 bis 18, vorzugsweise 1 bis 8, insbesondere 1 bis 6 Kohlenstoffatomen, einem Cycloalkylrest mit 5 bis 8 Kohlenstoffatomen, einen Aralkylrest mit 7 bis 12 Kohlenstoffatomen, oder den Rest $R^3—(OR^2)_n—$ bedeutet, $R^2$ einen Alkylenrest mit 2 bis 10, insbesondere 2 bis 4 Kohlenstoffatomen bezeichnet, $R^3$ für einen Alkylrest mit 1 bis 8, vorzugsweise 1 bis 4 Kohlenstoffatomen, einen Cycloalkylrest mit 5 bis 8 Kohlenstoffatomen oder einen Aralkylrest mit 7 bis 12 Kohlenstoffatomen steht, und n eine ganze Zahl von 1 bis 6, vorzugsweise 1, 2 oder 3 bedeutet. Die vorgenannten Reste können noch durch unter den Reaktionsbedingungen inerte Gruppen, z. B. Alkylgruppen oder Alkoxygruppen mit jeweils 1 bis 4 Kohlenstoffatomen, substituiert sein.

Harnstoff kann in stöchiometrischer Menge oder im Unterschuß, vorzugsweise in einer Menge von 1,5 bis 8, insbesondere 3 bis 6 Mol Ausgangsstoff II je Mol Harnstoff umgesetzt werden.

Es kommen z. B. die folgenden Alkohole als Ausgangsstoffe II in Frage : Benzyl-, Methyl-, Isopropyl-, Ethyl-, sekt.-Butyl-, Pentyl-, Cyclopentyl-, Phenylethyl-, Decyl-, n-Octyl-, n-Propyl-, n-Butyl-alkohol ; 2-Methoxypropyl-(1)-, 2-Ethoxypropyl-(1)-, 2-Methoxy-n-butyl-(1)-, 2-Ethoxy-n-butyl-(1)-, Methoxyethyloxyethyl-(1)-alkohol, Triethylenglykolmethylether ; bevorzugt sind Propanol, Butanol, Isobutanol, Hexanol, Heptanol, Cyclohexanol, Oktanol, 2-Ethylhexanol, Ethylenglykol-monomethylether oder -monoethylether.

Die Reaktion wird bei einer Temperatur von mindestens 140 °C, zweckmäßig 140 bis 250 °C, vorzugsweise von 160 bis 210 °C, unter Druck, kontinuierlich oder diskontinuierlich durchgeführt. Der Umsetzungsdruck beträgt mindestens 2 bar (Überdruck 1 bar), vorzugsweise 2 bis 31 (Überdruck 1 bis 30 bar), insbesondere 2 bis 12 bar. Im allgemeinen dient der Ausgangsstoff II gleichzeitig auch als Medium der Reaktion, gegebenenfalls kommen auch unter den Reaktionsbedingungen inerte, organische Lösungsmittel in Frage, z. B. aromatische Kohlenwasserstoffe wie Benzol, Toluol, Ethylbenzol, o-, m-, p-Xylol, Isopropylbenzol und entsprechende Gemische. Zweckmäßig verwendet man das Lösungsmittel in einer Menge von 50 bis 1 000 Gew.%, vorzugsweise von 100 bis 500 Gew.%, bezogen auf Ausgangsstoff II.

Als unter den Reaktionsbedingungen inerte Gase (Inertgas) werden zweckmäßig Edelgase wie Xenon, Argon, Neon, Helium ; bevorzugt Stickstoff und/oder Kohlendioxid ; und entsprechende Gemische ; verwendet. Bevorzugt ist eine Strömungsgeschwindigkeit des durch das Reaktionsgemisch geleiteten Inertgases von 1 bis 300, vorzugsweise 5 bis 100 Milliliter pro Stunde je Gramm Ausgangsstoff II.

Die Reaktion kann wie folgt durchgeführt werden : Ein Gemisch von Harnstoff und Ausgangsstoff II wird während 2 bis 10 Stunden bei der Reaktionstemperatur gehalten. Der bei der Reaktion entstehende Ammoniak wird durch Einleiten von Stickstoff aus der Reaktionslösung entfernt. Während der gesamten Reaktionszeit wird die eingestellte Reaktionstemperatur und das Sieden des Reaktionsgemisches durch entsprechende Druckveränderung, im allgemeinen Drucksenkung von 1 bis 10 bar Druck, gehalten. Dann wird das Reaktionsgemisch abgekühlt und filtriert. Aus dem Filtrat wird in üblicher Weise, z. B. durch Destillation, der Endstoff abgetrennt. Da Druck und Temperatur vom eingesetzten Alkohol abhängen, können die optimalen Werte leicht durch einen Vorversuch ermittelt werden. Der Druck wird zweckmäßig so eingestellt, daß das Gemisch unter schwachem Rückfluß siedet. Die Temperatur des Kondensators wird dabei so gewählt, daß der Alkohol fast vollständig kondensiert und möglichst wenig Ammoniak durch den Rückfluß zurückgeführt wird. Durch vorgenannte Verminderung des Druckes wird bei fortschreitender Umsetzung das Reaktionsgemisch ohne Änderung der Sumpftemperatur bei Rückfluß gehalten. Der Rückstand (Endstoff) besitzt allgemein eine so hohe Reinheit, daß er ohne zusätzliche Reinigung weiterverarbeitet werden kann (Beispiel 4).

Die nach dem Verfahren der Erfindung herstellbaren Verbindungen sind wertvolle Ausgangsstoffe für die Herstellung von Textilausrüstungsmitteln, Stabilisatoren, Weichmachern, Farbstoffen und Pflanzenschutzmitteln. Bezüglich der Verwendung wird auf die vorgenannten Veröffentlichungen verwiesen.

## Beispiel 1

In einem Rührautoklaven mit aufgesetzter Destillationskolonne wurde ein Gemisch aus 100 Gramm Harnstoff und 270 Gramm Ethylglykol auf 190 °C erhitzt und kontinuierlich ein schwacher Stickstoffstrom durch das Gemisch geleitet. Der Systemdruck wurde über ein Regelventil auf 7 bar eingestellt. Die aufsteigenden Dämpfe wurden bei 70 °C kondensiert und der gebildete Ammoniak über den Kopf der Kolonne abgetrennt. Das Gemisch wurde 7 Stunden bei 190 °C gehalten. Das Gemisch wurde durch Druckveränderung während der gesamten Reaktionszeit beim Sieden gehalten, wobei der Systemdruck während dieser Zeit kontinuierlich auf 3 bar abgesenkt wurde. Nach Beendigung der Umsetzung wurde das Reaktionsgemisch destillativ aufgearbeitet. Man erhält 212 Gramm (96 % der Theorie) reines Ethoxyethylcarbamat vom Kp 111-112 °C/0,2 mbar. Der Destillationsrückstand beträgt 2 Gramm.

Beispiel 2

In einem Rührreaktor mit aufgesetzter Destillationskolonne wurde ein Gemisch aus 100 Gramm Harnstoff und 368 Gramm Isobutanol auf 180 °C erhitzt und kontinuierlich ein schwacher Stickstoffstrom durch das Gemisch geleitet. Das Gemisch wurde durch Druckveränderung während der gesamten Reaktionszeit beim Sieden gehalten. Der Systemdruck wurde über ein Regelventil auf 7 bar eingestellt und die aufsteigenden Dämpfe bei 65 °C kondensiert, während der gebildete Ammoniak über den Kopf der Kolonne abgetrennt wurde. Das Reaktionsgemisch wurde 8 Stunden bei 180 °C gehalten und der Systemdruck während dieser Zeit kontinuierlich auf 3 bar abgesenkt. Nach Beendigung der Umsetzung wurde das überschüssige Isobutanol im Vakuum bei 100 °C/300 mbar abgetrennt. Man erhielt 191 Gramm (98 % der Theorie) Isobutylcarbamat mit einer Reinheit von 99 %. Der Schmelzpunkt des Endstoffs liegt bei 65-67 °C.

Beispiel 3

In einem Rührreaktor mit aufgesetzter Destillationskolonne wurde ein Gemisch aus 100 Gramm Harnstoff und 400 Gramm n-Butanol auf 190 °C erhitzt und kontinuierlich ein schwacher Stickstoffstrom durch das Gemisch geleitet. Das Gemisch wurde durch Druckveränderung während der gesamten Reaktionszeit beim Sieden gehalten. Der Systemdruck wurde über ein Regelventil auf 6 bar eingestellt und die aufsteigenden Dämpfe bei 70 °C kondensiert, während der gebildete Ammoniak über den Kopf der Kolonne abgetrennt wurde. Das Reaktionsgemisch wurde 6 Stunden bei 190 °C gehalten und der Systemdruck während dieser Zeit kontinuierlich auf 3 bar abgesenkt. Nach Beendigung der Umsetzung wurde das überschüssige Butanol im Vakuum bei 100 °C/300 mbar abgetrennt. Man erhielt 189 Gramm (97 % der Theorie) Butylcarbamat mit einer Reinheit von 99 %. Der Schmelzpunkt des Endstoffs liegt bei 51-53 °C.

Vergleichsbeispiel 1 (gemäß Beispiel 3 der DE-OS-2 459 765)

Ein Gemisch aus 100 Gramm Harnstoff, 270 Gramm Ethylglykol und 5 Gramm des unter der Bezeichnung Amberlyst 15 im Handel erhältlichen, entsprechend Beispiel 1a der DE-OS-2 459 765 behandelten, Nickel enthaltenden Austauscherharzes wurde in einem Rührreaktor unter Durchleiten eines schwachen Stickstoffstromes auf Rückflußtemperatur erhitzt (135 °C). Nach 7 Stunden stieg die Rückflußtemperatur auf 148 °C an. Bei dieser Temperatur wurde das Reaktionsgemisch 8 Stunden unter Durchleiten eines schwachen Stickstoffstromes gehalten. Nach Beendigung der Umsetzung wurde der Katalysator abgetrennt und das Reaktionsgemisch destillativ aufgearbeitet. Man erhielt 184 Gramm (83 % der Theorie) Ethoxyethylcarbamat vom Kp 112-113 °C/0,2 mbar. Der Destillationsrückstand betrug 17 Gramm. Das Reaktionsgemisch enthielt außerdem 420 ppm Nickel in gelöster Form.

Vergleichsbeispiel 2 (entsprechend DE-OS-2 459 765)

In einem Rührreaktor wurde ein Gemisch aus Gramm Harnstoff, 200 Gramm n-Butanol und 5 Gramm Katalysator (entsprechend Beispiel 1a der DE-OS-2 459 765) auf Rückflußtemperatur (135 °C) erhitzt. Nach 15 Stunden bei Rückflußtemperatur wurde der Katalysator abfiltriert und das Reaktionsgemisch, das 180 ppm gelöstes Nickel und entsprechen der gaschromatographischen Analyse 1,5 % Dibutylcarbonat enthielt, destillativ aufgearbeitet. Man erhielt 87 Teile (89 % der Theorie) Butylcarbamat vom Kp 109 bis 110 °C/20 mbar. Der Destillationsrückstand betrug 9 Gramm.

Beispiel 4 (Verwendung)

In einem Rührautoklaven wurde entsprechend Beispiel 1 der DE-AS-1 157 598 ein Gemisch aus 117 Gramm Roh-Butylcarbamat aus Beispiel 3, 200 Gramm Benzol, 20 Gramm Bortrifluoridetherat und 70 Gramm Isobuten auf 70 °C erhitzt und 8 Stunden bei dieser Temperatur gehalten. Nach Beendigung der Umsetzung wurde das Gemisch mit Wasser dreimal gewaschen und anschließend im Vakuum destilliert. Man erhielt 124 Gramm (71 % der Theorie) N-tert.-Butyl-butylcarbamat vom Kp 110 bis 111 °C/20 mbar.

**Patentansprüche**

1. Verfahren zur Herstellung von Carbamaten der Formel

$$R^1 - O - \overset{O}{\overset{\|}{C}} - NH_2 \qquad (I)$$

worin $R^1$ einen aliphatischen, cycloaliphatischen, araliphatischen Rest oder den Rest $R^3$—$(OR^2)_n$—

bedeutet, $R^2$ einen aliphatischen Rest mit mindestens 2 Kohlenstoffatomen bedeutet, $R^3$ für einen aliphatischen, cycloaliphatischen oder araliphatischen Rest steht, und n eine ganze Zahl bezeichnet, durch Umsetzung von Harnstoff mit Alkoholen, dadurch gekennzeichnet, daß man Harnstoff mit Alkoholen der Formel

$$R^1—OH \qquad (II)$$

worin $R^1$ die vorgenannte Bedeutung besitzt, bei einer Temperatur von mindestens 140 °C und einem Druck von mindestens 2 bar umsetzt, wobei Inertgas durch das Reaktionsgemisch geleitet und der Druck während der Umsetzung so eingestellt wird, daß das Reaktionsgemisch siedet.

2. Verfahren nach Anspruch 1, dadurch gekennzeichnet, daß man die Umsetzung ohne Verwendung eines Katalysators durchführt.

## Claims

1. A process for the preparation of a carbamate of the formula

$$R^1 - O - \overset{O}{\overset{\parallel}{C}} - NH_2 \qquad (I)$$

where $R^1$ is an aliphatic, cycloaliphatic or araliphatic radical or $R^3—(OR^2)_n—$, $R^2$ is an aliphatic radical of not less than 2 carbon atoms, $R^3$ is an aliphatic, cycloaliphatic or araliphatic radical, and n is an integer, by reacting urea with an alcohol, wherein urea is reacted with an alcohol of the formula

$$R^1—OH \qquad (II)$$

where $R^1$ has the above meaning, at not less than 140 °C and under not less than 2 bar, an inert gas being passed through the reaction mixture and the pressure during the reaction being so set that the reaction mixture boils.

2. A process as claimed in claim 1, wherein the reaction is carried out in the absence of a catalyst.

## Revendications

1. Procédé pour la préparation de carbamates de formule

$$R^1 - O - \overset{O}{\overset{\parallel}{C}} - NH_2 \qquad (I)$$

dans laquelle $R^1$ représente un radical aliphatique, cycloaliphatique, araliphatique ou le radical $R^3—(OR^2)_n$, $R^2$ étant un radical aliphatique comportant au moins 2 atomes de carbone, $R^3$ étant mis pour un radical aliphatique, cycloaliphatique ou araliphatique et n étant un nombre entier, par réaction d'urée avec des alcools, caractérisé en ce qu'on fait réagir l'urée avec des alcools de formule

$$R^1—OH \qquad (II)$$

dans laquelle $R^1$ a la signification donnée ci-dessus, à une température d'au moins 140 °C et sous une pression d'au moins 2 bars, un gaz inerte étant envoyé à travers le mélange réactionnel et la pression étant réglée pendant la réaction de telle manière que le mélange réactionnel soit en ébullition.

2. Procédé selon la revendication 1, caractérisé en ce qu'on mène la réaction sans utilisation d'un catalyseur.